# DEMANDE DE BREVET EUROPEEN

(11) **EP 2 039 289 A2**
(43) Date de publication de la demande: **25.03.2009**
(21) Numéro de dépôt: 08163459.4
(22) Date de dépôt: 02.09.2008
(51) Int. Cl.: A61B 5/00, H01Q 1/27

(54) **Système de surveillance médicale d'une personne**

(30) Priorité: 18.09.2007 FR 0706568
(71) Demandeur: Tecknisolar Seni, 35400 Saint-Malo (FR); Societe De Confection Balsan, 36130 Montierchaume (FR)
(72) Inventeur: BARGUIRDJIAN, Pascal, 35400, SAINT-MALO (FR)
(74) Mandataire: Maillet, Alain

(57) **Abrégé**

La présente invention concerne un système de surveillance (1) comprenant un habit (10) destiné à être porté par une personne incorporant au moins un capteur de grandeur physique (11) fournissant des signaux de mesure et un dispositif de traitement électronique (14) aptes à traiter lesdits signaux de mesure issus du ou des capteurs de grandeurs physiques (11).

Selon l'invention, le système de surveillance comporte un premier ensemble de connexion (12) qui est disposé sur ou dans ledit habit (10) et auquel est relié ledit ou lesdits capteurs de grandeurs physiques (11). En outre, ledit dispositif de traitement électronique (14) est intégré à un second ensemble de connexion (13) apte à être fixé et à collaborer de manière temporaire avec ledit premier ensemble de connexion (12) pour assurer la connexion électrique du ou des capteurs de grandeurs physiques (11) audit dispositif de traitement électronique (14).

## Description

La présente invention concerne un système de surveillance médicale d'une personne. Un tel système a pour but de mesurer en permanence les valeurs prises par des grandeurs physiques prédéfinies liées à l'état de santé de la personne à surveiller et de traiter lesdites valeurs afin, par exemple, d'émettre une alerte si ces grandeurs physiques prennent des valeurs qui peuvent être considérées comme non souhaitables ou dangereuses vis-à-vis de la santé de ladite personne.

Lorsqu'elle est en crise de sa maladie, une personne malade a son état qui se modifie, ce qui se traduit généralement par une modification des valeurs prises par certaines grandeurs physiques, telles que la température, le taux d'humidité, etc. Ces modifications peuvent faire l'objet de mesures au moyen de capteurs de grandeurs physiques appropriés. Ainsi, la personne diabétique qui vit une crise de diabète profond et qui risque ainsi de tomber dans le coma, transpire beaucoup et sa température corporelle chute. Le taux d'humidité et la température du corps du malade sont deux grandeurs physiques qu'il est possible de mesurer dans le but, par exemple, de détecter un risque d'occurrence d'un état de coma et d'alerter les médecins ou une personne du voisinage qui peuvent agir à temps pour sauver le malade.

Ainsi, il a déjà été proposé des systèmes de surveillance qui comportent des capteurs de grandeurs physiques pour la mesure de grandeurs physiques liées à l'état de santé d'un malade et des dispositifs de traitement électronique des signaux de mesure générés par lesdits capteurs de grandeurs physiques, traitement consistant par exemple à comparer les valeurs prises par les signaux de mesure à des valeurs seuils et à déclencher une alarme en cas de dépassement desdites valeurs seuils. Ces systèmes de surveillance sont soit enfermés dans la doublure d'un habit tel qu'une veste, une ceinture pectorale, un bracelet, etc., soit seulement liés à cet habit. On appelle "habit" tout objet que peut porter une personne en l'habillant, en la couvrant même de manière très partielle. L'inconvénient rencontré par ce type de système de surveillance est lié à l'habit qu'ils utilisent. En effet, que ce soit une veste, une ceinture ou un bracelet, s'il est utilisé de manière fréquente, comme cela doit être le cas pour le suivi permanent d'une personne, il doit aussi être régulièrement lavé. Or, le lavage d'un tel habit suppose de prendre des précautions particulières du fait de la présence des dispositifs de traitement électronique qui peuvent être endommagés par la présence de l'eau de lavage mais aussi par les sollicitations mécaniques dues, par exemple, au tambour ou autre de la machine à laver.

La présente invention a donc pour but de proposer un système de surveillance médicale d'une personne qui soit d'une structure telle que le lavage de l'habit qu'utilise ledit système puisse être nettoyé sans risques de dégradations des dispositifs de traitement électronique qui le composent.

A cet effet, la présente invention concerne un système de surveillance comprenant un habit destiné à être porté par une personne incorporant au moins un capteur de grandeur physique fournissant des signaux de mesure et un dispositif de traitement électronique aptes à traiter lesdits signaux de mesure issus du ou des capteurs de grandeurs physiques.

Comporte un premier ensemble de connexion qui est disposé sur ou dans ledit habit et auquel est relié ledit ou lesdits capteurs de grandeurs physiques et en ce que ledit dispositif de traitement électronique est intégré à un second ensemble de connexion apte à être fixé et à collaborer de manière temporaire avec ledit premier ensemble de connexion pour assurer la connexion électrique du ou des capteurs de grandeurs physiques audit dispositif de traitement électronique.

Selon une caractéristique de l'invention, le dispositif de traitement électronique comporte une unité électronique de traitement apte à recevoir les signaux de mesure générés par le ou les capteurs de grandeurs physiques, à interpréter lesdits signaux de mesure et à générer un signal d'alerte lorsque lesdits signaux de mesure correspondent à un état anormal à surveiller.

Selon une autre caractéristique de l'invention, le dispositif de traitement électronique comporte une unité électronique de traitement apte à recevoir les signaux de mesure générés par le ou les capteurs de grandeurs physiques, à interpréter lesdits signaux de mesure et à transmettre à une unité de contrôle distante des signaux issus de ladite interprétation.

Selon une autre caractéristique de l'invention, pour transmettre lesdits signaux à ladite unité de contrôle distante, le second ensemble de connexion comprend un émetteur équipé d'une antenne pour pouvoir émettre lesdits signaux sous forme de signaux radiofréquences.

Selon une autre caractéristique de l'invention, ladite antenne est positionnée sur ou dans ledit vêtement et est reliée à un premier ensemble de connexion disposé sur ou dans ledit habit, ledit émetteur étant relié à un second ensemble de connexion apte à être fixé et à collaborer de manière temporaire avec ledit premier ensemble de connexion pour assurer la connexion électrique de ladite antenne audit émetteur.

Selon une autre caractéristique de l'invention, ladite antenne est un élément métallique du vêtement, tel qu'un bouton.

Selon une autre caractéristique de l'invention, ladite antenne est constituée d'un ruban électriquement conducteur du premier ensemble de connexion apte à coopérer avec un ruban électriquement conducteur dudit second ensemble de connexion pour sa connexion audit émetteur.

Selon une autre caractéristique de l'invention, le premier ensemble de connexion et le second ensemble de connexion comprennent des paires de rubans électriquement conducteurs constitués de rubans auto-agrippants métallisés.

Selon une autre caractéristique de l'invention, lesdits capteurs sont des composants passifs étanches à l'immersion.

Selon une autre caractéristique de l'invention, le système de surveillance comporte un capteur de température et un capteur d'humidité en tant que capteurs de grandeurs physiques.

Selon une autre caractéristique de l'invention, l'interprétation effectuée par ladite unité de traitement électronique consiste à comparer la valeur des signaux issus des capteurs de grandeurs physiques à des valeurs seuils prédéterminées.

Selon une autre caractéristique de l'invention, le dispositif de traitement électronique est apte à affecter les paires de rubans électriquement conducteurs du second ensemble de connexion à la réception de signaux de mesure déterminés.

Selon une autre caractéristique de l'invention, le second ensemble de connexion comporte un émetteur apte à permettre l'émission sous forme de signaux radiofréquences de données issues du dispositif de traitement électronique à destination d'un récepteur d'une unité de contrôle distante prévue pour émettre des signaux d'alerte.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
la Fig. 1 représente un exemple de réalisation d'un système de surveillance selon l'invention,
la Fig. 2 représente les parties fonctionnelles du système de surveillance selon un premier mode de réalisation de l'invention, et
la Fig. 3 représente les parties fonctionnelles du système de surveillance selon un second mode de réalisation de l'invention.

Un système de surveillance médicale 1 selon la présente invention comporte un habit 10, par exemple un vêtement tel qu'un pyjama, dans lequel sont logés des capteurs de grandeurs physiques 11, avantageusement du type passif étanche à l'immersion. Les capteurs de grandeurs physiques 11 utilisés sont par exemple un capteur de température et un capteur d'humidité, notamment lorsque le système de surveillance est utilisé pour la surveillance d'un malade diabétique. Un capteur de rythme cardiaque peut également être utilisé. Ces capteurs de grandeurs physiques 11 sont soit disposés sur la paroi intérieure du vêtement 10, soit logés dans sa doublure à des endroits appropriés de celui-ci, par exemple au droit du sternum ou des aisselles, pour que les mesures faites soient les plus représentatives possibles de l'état de santé du malade à surveiller. Un habit 10 selon l'invention peut aussi bien être une veste, un tee-shirt, un pyjama, une ceinture, une casquette, etc.

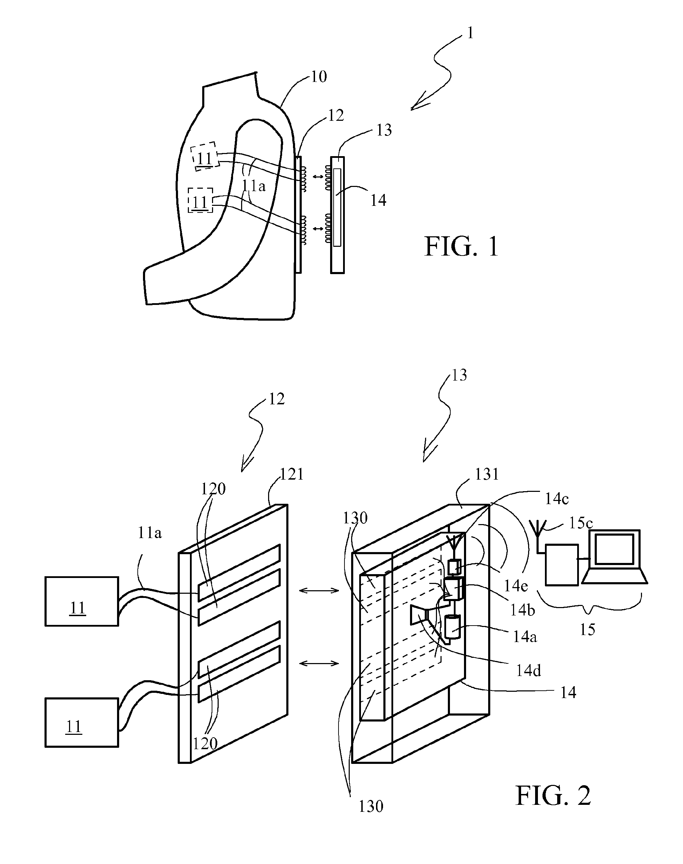

Dans un exemple de réalisation de l'invention représenté à la Fig.1, les capteurs de grandeurs physiques 11 sont reliés au travers de l'habit 10 par l'intermédiaire de liaisons 11a, de préférence filaires, à un premier ensemble de connexion 12 positionné en surface de l'habit 10.

Le système de surveillance médicale selon la présente invention représenté à la Fig. 1 comporte encore un second ensemble de connexion 13 qui est apte à être fixé de manière facilement démontable et temporaire sur le premier ensemble de connexion 12 et qui est prévu pour pouvoir coopérer avec le premier ensemble de connexion 12, comme on le verra par la suite. Le second ensemble de connexion 13 inclut un dispositif de traitement électronique 14.

Ainsi que cela est représenté à la Fig. 2, selon un mode de réalisation de l'invention, le premier ensemble de connexion 12 comporte un support 121 sur lequel est montée au moins une paire de rubans électriquement conducteurs 120, chaque ruban d'une paire 120 étant relié à une borne d'un capteur de grandeurs physiques 11 par une liaison 11a telle qu'un conducteur électrique : fil électrique ou bande métallique ou métallisée par des particules métalliques ou aurifères. De même, le second ensemble de connexion 13 comporte également un support 131 sur lequel est monté le même nombre de paires de rubans électriquement conducteurs 130 que pour le premier ensemble de connexion 12 de manière à ce que cette paire ou ces paires de rubans électriquement conducteurs 130 puissent entrer en contact mécanique et électrique avec une paire ou des paires de rubans électriquement conducteurs 120 du premier ensemble de connexion 12 et ainsi assurer une ou des connexion(s) électrique(s) entre un ou des capteur(s) de grandeurs physiques 11 et le dispositif de traitement électronique 14.

La connexion entre le premier ensemble de connexion 12 et le second ensemble de connexion 13 permet la transmission des données issues des capteurs de grandeurs physiques 11 au dispositif de traitement électronique 14.

Lorsqu'on désire laver l'habit 10, le second ensemble de connexion 13 est séparé du premier ensemble de connexion 12 et ainsi de l'habit 10. Les capteurs de grandeurs physiques 11, tels que les capteurs de température et les capteurs d'humidité, notamment du fait que ce sont des composants passifs étanches à l'immersion, ne sont pas dégradés par l'eau de lavage ou des sollicitations mécaniques dues au lavage. Du fait de la séparation, le dispositif de traitement électronique 14 est temporairement éloigné de l'habit 10 et peut donc être isolé.

Lorsque l'habit 10 est porté par le malade, le second ensemble de connexion 13 est connecté au premier ensemble de connexion 12 ce qui assure la connexion électrique du ou des capteurs de grandeurs physiques 11 avec le dispositif de traitement électronique 14.

De manière avantageuse, chaque paire de rubans électriquement conducteurs 120 ou 130 est constituée de deux rubans auto-agrippants, par exemple de deux rubans du type connu sous le terme de velcro ®, qui ont été métallisés par recouvrement d'une matière métallique, par exemple constituée de poudre d'aluminium ou de poudre aurifère. Par exemple, si les rubans auto-agrippants constituant les paires de rubans électriquement conducteurs 120 sont constitués de crochets, les rubans auto-agrippants constituant les paires de rubans électriquement conducteurs 130 sont constitués eux, de boucles prévues pour recevoir des crochets. Ainsi, lorsqu'une paire de rubans électriquement conducteurs 120 est mise en contact avec une paire de rubans électriquement conducteurs 130, il y a création à la fois d'une liaison électrique permettant la transmission des signaux délivrés par des capteurs de grandeurs physiques 11 au dispositif de traitement électronique 14 mais aussi d'une liaison mécanique entre les paires de rubans électriquement conducteurs 120 et 130.

Le dispositif de traitement électronique 14 est incorporé dans le support 131 et est relié aux paires de rubans électriquement conducteurs 130 de manière à recevoir, lorsque lesdits deux ensembles de connexion 12 et 13 sont connectés l'un à l'autre, les signaux de mesure délivrés par les capteurs de grandeurs physiques 11. Le dispositif de traitement électronique 14 comporte, par exemple, un moyen tel qu'une pile ou une batterie rechargeable 14a pour assurer son alimentation électrique, une unité électronique de traitement 14b.

L'unité électronique de traitement 14b permet notamment de traiter les signaux de mesure reçus des capteurs de grandeurs physiques 11.

Ce traitement consiste, par exemple, à interpréter les signaux de mesure reçus et à émettre un signal d'alerte sonore et/ou lumineux lorsque les signaux de mesure sont représentatifs d'un état anormal de la personne portant le vêtement.

Ce traitement pourrait également consister, par exemple, à transformer ces signaux sous forme de données exploitables par un logiciel approprié et à les transmettre ensuite à une unité de contrôle distante 15 que comporterait alors le système de surveillance. L'unité de contrôle distante 15 est de préférence un ordinateur équipé d'un récepteur pourvu d'une antenne 15c. De manière alternative, ce traitement pourrait également consister à interpréter les signaux de mesure reçus de manière à ne transmettre par la suite à l'unité de contrôle distante 15 qu'un simple signal d'alerte.

Dans le cas où le système de surveillance comporte ladite unité de contrôle distant 15 et selon un mode de réalisation de l'invention, le dispositif de traitement électronique 14 comporte encore un émetteur 14e pourvu d'une antenne 14c qui sont utilisés pour l'émission sous forme de signaux radiofréquences de données délivrées par l'unité électronique de traitement 14b vers un récepteur pourvu d'une antenne 15c que comporte l'unité de contrôle distante 15. Ces données peuvent, soit correspondre à la transcription des signaux de mesure, soit être des données binaires correspondant à un état d'alerte : 1 correspondant à une alerte, 0 à une non-alerte.

On notera que la transmission des données de l'unité de traitement électronique 14b à l'unité de contrôle distante 15 pourrait également être assurée, plutôt que par une liaison radiofréquence comme cela vient d'être décrit, par une liaison filaire ou autre. Chacune de ces unités 14b et 15 comporterait alors les moyens nécessaires à assurer cette liaison.

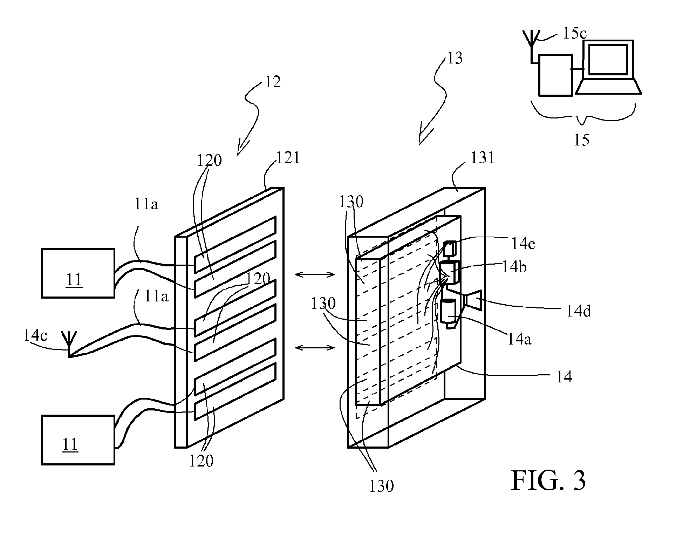

Dans un second mode de réalisation de l'invention représenté à la Fig. 3, l'antenne 14c est avantageusement positionnée dans ou sur le vêtement 10. De manière préférée, cette antenne 14c peut être constituée par tout élément métallique du vêtement susceptible de jouer le rôle d'antenne et notamment par un bouton métallique du vêtement 10. Les autres éléments de l'invention sont identiques dans leur structure et dans leur fonctionnement à ce qui a été décrit en relation avec le premier mode de réalisation.

Dans un troisième mode de réalisation de l'invention, non représenté, un des rubans électriquement conducteurs 120 est utilisé en tant qu'antenne 14c. Il a alors des dimensions qui correspondent à une bonne émission dans la longueur d'onde d'émission considérée.

Ainsi, à l'instar des capteurs de grandeurs physiques 11 décrits précédemment, l'antenne 14c est reliée par l'intermédiaire d'une liaison 11a, de préférence filaire, à une paire de rubans électriquement conducteurs 120 du premier ensemble de connexion 12. Sur le second ensemble de connexion 13, est prévue une paire de rubans électriquement conducteurs 130 apte à entrer en contact électrique avec la paire de rubans électriquement conducteurs 120 du premier ensemble de connexion 12 à laquelle est reliée l'antenne 14c. Cette paire de rubans électriquement conducteurs 130 est par ailleurs reliée à l'unité de traitement électronique 14b du dispositif de traitement électronique 14.

Ainsi, les signaux de données délivrés par l'unité électronique de traitement 14b sont délivrés à l'antenne 14c par l'intermédiaire de la paire de rubans électriquement conducteurs 130 du second ensemble de connexion 13, puis de la paire de rubans électriquement conducteurs 120 du premier ensemble de connexion 12. L'antenne 14c transmet alors ces signaux à l'unité de contrôle distant 15.

Selon un exemple de réalisation, deux capteurs de grandeurs physiques 11 sont prévus : l'un de température et l'autre d'humidité. Les signaux de mesure délivrés par ces capteurs de grandeurs physiques 11 sont transmis via le premier ensemble de connexion 12 et le second ensemble de connexion 13 à l'unité électronique de traitement 14b du dispositif de traitement 14. Par exemple, ladite unité de traitement 14b convertit les signaux de mesure issus des capteurs de grandeurs physiques en données numériques qui sont ensuite traitées, par exemple, pour effectuer une comparaison entre les valeurs des données ainsi obtenues et des valeurs seuils prédéterminées de ces mêmes données. Dans le cas où cette comparaison ne fait pas apparaître de dépassement au-delà de la valeur seuil d'humidité utilisée en référence ou de dépassement en deçà de la valeur seuil de température utilisée en référence, les données obtenues des capteurs de grandeurs physiques 11 sont considérées comme normales et aucune procédure d'alerte n'est déclenchée.

Dans le cas contraire, autrement dit, si la valeur relative aux données d'humidité est supérieure à la valeur seuil d'humidité ou si la valeur relative aux données de température est inférieure à la valeur seuil de température, alors une procédure d'alerte est déclenchée. Cela peut notamment être le cas lors d'une crise de diabète pendant laquelle la personne transpire énormément et voit sa température corporelle chuter.

La procédure d'alerte consiste à ce que le dispositif 14 émette lui-même un signal d'alerte sonore et/ou lumineux.

La procédure d'alerte pourrait également consister à transmettre à l'unité de contrôle distante 15 un signal radiofréquence qui, après réception, engendrera des signaux, par exemple sonores et/ou lumineux, d'alerte.

De manière optionnelle et avantageuse, l'unité électronique de traitement 14b détecte l'existence d'une liaison électrique entre les capteurs de grandeurs physiques 11 et le dispositif de traitement électronique 14. En outre, en cas de rupture de cette liaison électrique, due à la séparation du premier ensemble de connexion 12 du second ensemble de connexion 13, l'unité électronique de traitement 14b se met en veille entraînant ainsi une réduction de l'énergie électrique consommée. Inversement, la création de la liaison entre les capteurs de grandeurs physiques 11 et l'unité électronique de traitement 14b engendre la mise hors veille de cette dernière.

Egalement de manière optionnelle et avantageuse, l'unité électronique de traitement 14b est apte à affecter les paires de rubans électriquement conducteurs 130 du second ensemble de connexion 13 à la réception de signaux de mesure déterminés. Ainsi lorsqu'une paire de rubans électriquement conducteurs 120 portant des signaux de mesure de température est branchée sur une paire de rubans électriquement conducteurs 130, cette paire de rubans électriquement conducteurs 130 est automatiquement affectée par l'unité électronique de traitement 14b à la réception de données de température.

## Revendications

1. Système de surveillance (1) comprenant un habit (10) destiné à être porté par une personne incorporant au moins un capteur de grandeur physique (11) fournissant des signaux de mesure et un dispositif de traitement électronique (14) aptes à traiter lesdits signaux de mesure issus du ou des capteurs de grandeurs physiques (11), **caractérisé en ce qu'**il comporte un premier ensemble de connexion (12) qui est disposé sur ou dans ledit habit (10) et auquel est relié ledit ou lesdits capteurs de grandeurs physiques (11) et **en ce que** ledit dispositif de traitement électronique (14) est intégré à un second ensemble de connexion (13) apte à être fixé et à collaborer de manière temporaire avec ledit premier ensemble de connexion (12) pour assurer la connexion électrique du ou des capteurs de grandeurs physiques (11) audit dispositif de traitement électronique (14).

2. Système de surveillance selon une des revendications précédentes, **caractérisé en ce que** le premier ensemble de connexion (12) et le second ensemble de connexion (13) comprennent des paires de rubans électriquement conducteurs (120, 130) constitués de rubans auto-agrippants métallisés.

3. Système de surveillance selon l'une des revendications 1 à 2, **caractérisé en ce que** le dispositif de traitement électronique (14) comporte une unité électronique de traitement (14b) apte à recevoir les signaux de mesure générés par le ou les capteurs de grandeurs physiques (11), à interpréter lesdits signaux de mesure en comparant la valeur des signaux issus des capteurs de grandeurs physiques (11) à des valeurs seuils prédéterminées et à générer un signal d'alerte lorsque lesdits signaux de mesure correspondent à un état anormal à surveiller.

4. Système de surveillance selon l'une des revendications 1 à 2, **caractérisé en ce que** le dispositif de traitement électronique (14) comporte une unité électronique de traitement (14b) apte à recevoir les signaux de mesure générés par le ou les capteurs de grandeurs physiques (11), à interpréter lesdits signaux de mesure en comparant la valeur des signaux issus des capteurs de grandeurs physiques (11) à des valeurs seuils prédéterminées et à transmettre à une unité de contrôle distante (15) des signaux issus de ladite interprétation.

5. Système de surveillance selon la revendication 4, **caractérisé en ce que**, pour transmettre lesdits signaux à ladite unité de contrôle distante (15), le second ensemble de connexion (13) comprend un émetteur (14e) lequel est relié à une antenne (14c) pour pouvoir émettre lesdits signaux sous forme de signaux radiofréquences.

6. Système de surveillance selon la revendication 5, **caractérisé en ce que** ladite antenne (14c) est positionnée sur ou dans ledit vêtement (10) et est reliée à un premier ensemble de connexion (12) disposé sur ou dans ledit habit (10), ledit émetteur (14e) étant relié à un second ensemble de connexion (13) apte à être fixé et à collaborer de manière temporaire avec ledit premier ensemble de connexion (12) pour assurer la connexion électrique de ladite antenne (14c) audit émetteur (14e).

7. Système de surveillance selon la revendication 6, **caractérisé en ce que** ladite antenne (14c) est un élément métallique du vêtement (10), tel qu'un bouton.

8. Système de surveillance selon la revendication 5, **caractérisé en ce que** ladite antenne (14c) est constituée d'un ruban électriquement conducteur (120) du premier ensemble de connexion (12) apte à coopérer avec un ruban électriquement conducteur (130) dudit second ensemble de connexion (13) pour sa connexion audit émetteur (14e).

9. Système de surveillance selon une des revendications précédentes, **caractérisé en ce que** lesdits capteurs sont des composants passifs étanches à l'immersion.

10. Système de surveillance selon l'une des revendications précédentes, **caractérisé en ce qu'**il comporte un capteur de température et un capteur d'humidité en tant que capteurs de grandeurs physiques (11).

11. Système de surveillance selon une des revendications précédentes, **caractérisé en ce que** le dispositif de traitement électronique (14) comporte des moyens d'affectation des paires de rubans électriquement conducteurs (130) du second ensemble de connexion (13) à la réception de signaux de mesure déterminés.

12. Système de surveillance selon l'une des revendications précédentes, **caractérisé en ce qu'**il comporte une unité de contrôle distante (15) prévue pour émettre des signaux d'alerte.
